# EUROPEAN PATENT SPECIFICATION

(11) **EP 4 525 790 B1**
(45) Date of publication and mention of the grant of the patent: **18.03.2026**
(21) Application number: 23724319.1
(22) Date of filing: 12.05.2023
(51) Int. Cl.: A61F 4/00

(54) **HAND-HELD GRIP AID DEVICE FOR HAND INSTRUMENT MANIPULATION**
HANDHALTBARE GRIFFHILFEVORRICHTUNG ZUR HANDHABUNG VON HANDINSTRUMENTEN
DISPOSITIF D'AIDE À LA PRÉHENSION TENU À LA MAIN POUR MANIPULATION D'INSTRUMENT À MAIN

(30) Priority: 17.05.2022 DK PA202200463
(43) Date of publication of application: 26.03.2025
(73) Proprietor: Manigrip APS, 7100 Vejle (DK)
(72) Inventor: PEDERSEN, Henrikke Kylén, 8230 Åbyhøj (DK); ANDERSEN, Thomas Solgaard, 7100 Vejle (DK); AAGAARD, Erik, 9560 Hadsund (DK); JOHANSEN, Kim Ivan, 9550 Mariager (DK)
(74) Representative: Larsen & Birkeholm A/S
(86) International application number: PCT/EP2023/062705
(87) International publication number: WO 2023/222526

(56) References cited:
- US-A- 4 711 482
- US-A- 5 131 116
- US-A1- 2020 306 951

## Description

### Technical field of the invention

The present invention relates to hand-held grip aid devices for hand instrument manipulation.

### Background of the invention

Hand-held grip aid devices for hand instrument manipulation are used to facilitate the gripping of articles of daily use for people with restricted mobility of the hands, such as for persons with hand injuries or illnesses causing reduced mobility of the hands.

The problem with existing grip aids on the market today, is that they are often developed for only one shape of hand instrument, such as for example a flat-handled fork, or a round-handled pencil.

US20080083306 discloses an apparatus for gripping a tool shaft and includes a tubular handle having an opening for receiving a tool shaft; an air bladder disposed within the tubular handle for selectively constricting the opening; and a valve in pneumatic communication with the air bladder for inflating the air bladder, thereby causing the air bladder to grip a tool shaft inserted therethrough.

US 2020306951 discloses a device that will hold an implement for manually impaired persons.

Hence, it is an object of the present invention to provide a new type of gripping aid, which allows flexible use of the gripping aid with different shapes of hand instruments.

### Description of the invention

The present invention relates to a hand-held grip aid as set out in the claims.

In one or more embodiments, the (twisting) mechanism comprises:
- a ring- or tube-shaped drive shaft rotatably mounted in said housing; and
- an electric motor unit positioned within said housing, and indirectly, e.g., through a gear, or directly, adapted to transfer a torque to said drive shaft.

In one or more embodiments, the grip aid device comprises an inner housing and an outer housing. Preferably, the holder is integrated into the inner housing, e.g., may the inner housing be part of the holder. The outer housing may in some embodiments be configured as a cover releasably fastened to the inner housing, and wherein the outer housing comprises an opening leading into the lumen of the holder, e.g., one or more, preferably one in each end of the outer housing, if the lumen extends all the way through the inner housing.

In one or more embodiments, the drive shaft and said housing comprises a stop mechanism adapted for allowing said drive shaft to rotate a predefined number of degrees around its axis of rotation. The stop mechanism comprises a first stop pin extending outwardly from the drive shaft, and preferably a second stop pin attached to said housing.

In one or more embodiments, the drive shaft comprises a lumen, and wherein the tubular lining is attached to the lumen of the drive shaft. Several drive shafts and motor units may be present, but it is preferred that only a single drive shaft and motor unit is present, as also shown in the examples below. A single motor unit may also be operably connected to multiple drive shafts, such as two drive shafts. The tubular lining may be attached to the drive shaft(s) e.g., by adhesion, clamping, bolting, riveting, or the like, and may be attached to the inner housing at multiple sites. Preferably, a drive shaft is positioned centrally along the length of said grip aid device, and more preferably a single drive shaft is positioned centrally along the length of said grip aid device. In the latter situation, the tubular lining may be attached to the inner housing at the ends of the grip aid device.

In one or more embodiments, the lumen of the holder extends along the entire length of grip aid device or at least along the entire length of the holder. Preferably, the lumen of the holder is open-ended, thereby allowing the handle of a hand instrument to be inserted there through.

In one or more embodiments, the lumen of said holder extends along the entire length of said holder, wherein the lumen of said holder is open-ended, and wherein the outer housing comprises openings leading into each of the lumen's ends, thereby allowing the handle of a hand instrument to be inserted there through.

In one or more embodiments, the tubular lining is made in one piece. In order for the tubular lining to be flexible and twistable, it should preferably be made from an elastomeric material, such as rubber, polyurethane, natural rubber, neoprene rubber, nitrile rubber, silicone, synthetic rubber, such as polyisoprene, polybutadiene, polychloroprene, butyl rubber, styrene-butadiene co-polymers, acrylonitrile-butadiene-styrene co-polymers, EPDM (ethylene-propylene-diene) terpolymers, and the like or the like.

As used in the specification and the appended claims, the singular forms "a", "an", and "the" include plural referents unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about", it will be understood that the particular value forms another embodiment.

### Brief description of the figures

Figure 1 shows a grip aid device in accordance with various embodiments of the invention, where the outer housing is made transparent.
Figure 2 shows a grip aid device in accordance with various embodiments of the invention.
Figure 3 shows a grip aid device in accordance with various embodiments of the invention positioned in a charging dock.
Figure 4 shows a grip aid device in accordance with various embodiments of the invention, where the outer housing is removed.

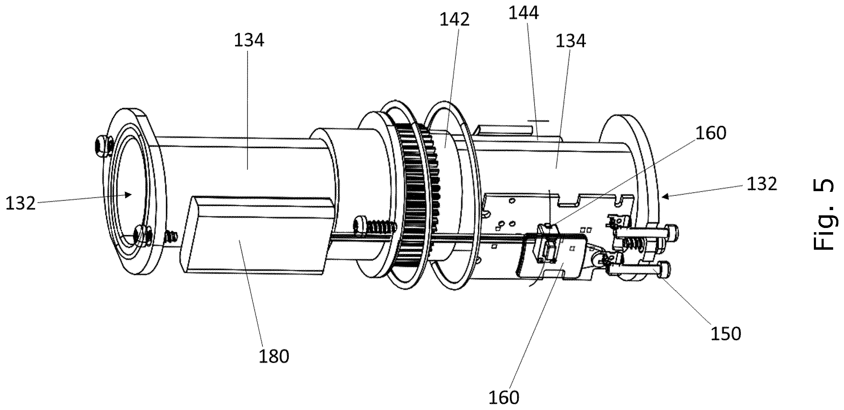

Figure 5 shows a grip aid device in accordance with various embodiments of the invention, where a part of the inner housing is removed.
Figure 6 shows a grip aid device in accordance with various embodiments of the invention, where a part of the inner housing is removed.
Figure 7 shows a drive mechanism adapted for twisting the holder of the grip aid device in accordance with various embodiments of the invention.
Figure 8 shows a part of an inner housing in accordance with various embodiments of the invention.
Figure 9 shows a lining in accordance with various embodiments of the invention.
Figure 10 shows a grip aid device in accordance with various embodiments of the invention, where a part of the inner housing is removed, and the lining is twisted.
Figure 11 shows a grip aid device in accordance with various embodiments of the invention, where a part of the inner housing is removed, and the lining is twisted around a hand instrument.

### Detailed description of the invention

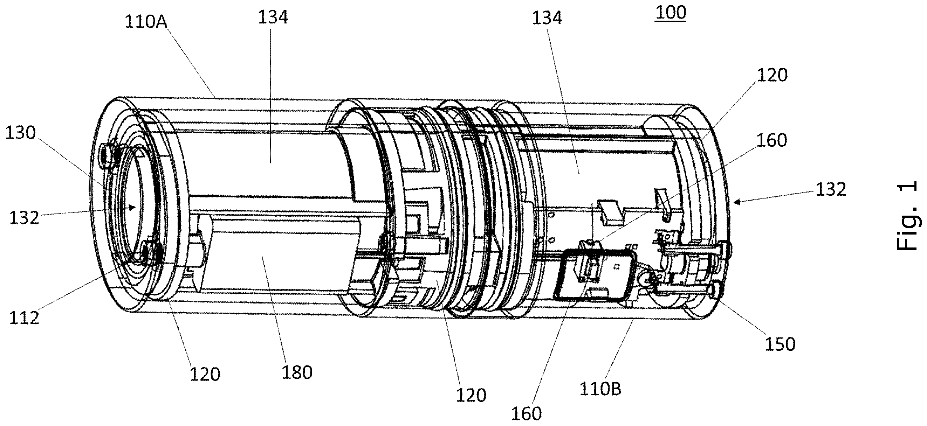

Figure 1 shows a grip aid device 100 in accordance with various embodiments of the invention, where the outer casing 110 is made transparent for a better view of the individual components. A handle is integrated into the outer housing/casing 110, i.e., the shape of the outer housing/casing 110 allows a user to hold it with her or his hand(s). The outer housing/casing 110 comprises two parts 110A, 110B that are releasably fastened to an inner housing/casing 120 and to one another. This configuration allows a user to remove the outer casing 110 for cleaning, e.g., in a dishwasher. Furthermore, it allows the user to change the outer casing/housing 110 when worn or simply if another appearance or grip function (shape or colour) is wanted. The grip aid device 100 may comprise different shapes of outer casings 110 (e.g., a kit of outer housing/casings, e.g., with different grip functions) for different types of grips. In general, the inner housing/casing 120 may be configured to cover parts of the grip aid device and/or configured as a chassis for supporting and/or holding parts of the grip aid device.

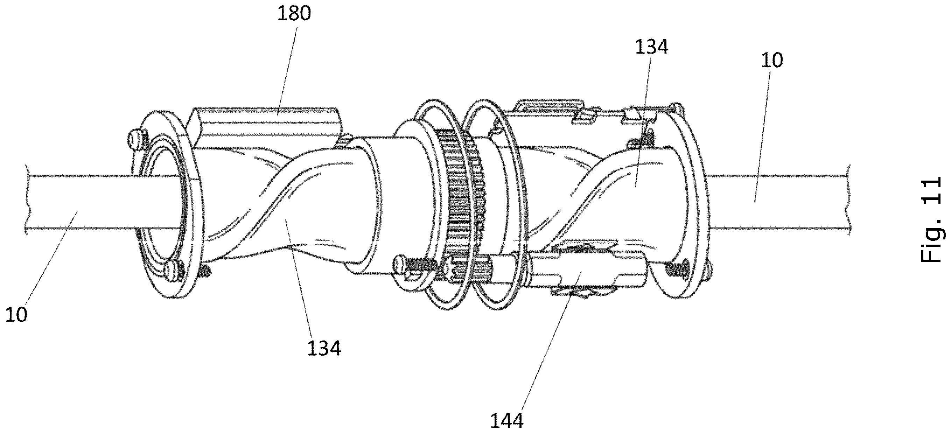

Apart from the outer 110 and inner 120 housing/casings, the grip aid device 100 also comprises a holder 130 integrated into the inner housing 120 and adapted for receiving the handle of a hand instrument as e.g., seen in Figure 11.

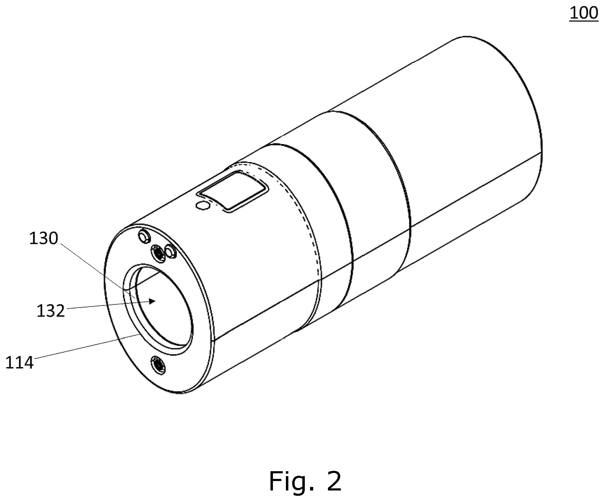

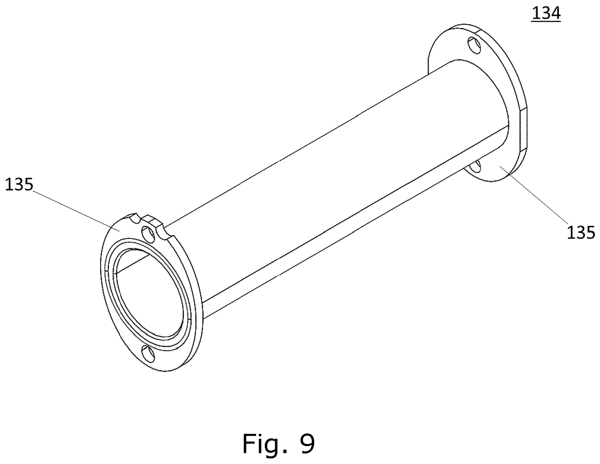

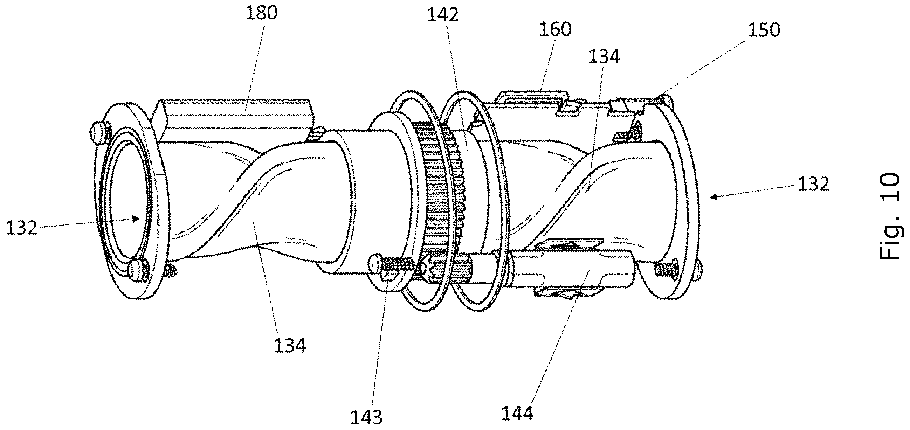

The holder 130 comprises a lumen 132 adapted for being in an open, relatively wide, configuration (as e.g., seen in Figure 2), and in a closed, relatively narrow, configuration (as e.g., seen in Figure 10). The size/width of the lumen is regulated by acting on, i.e., twisting, a flexible tubular lining 134 (see e.g., Figure 9), also being part of the holder 130. As the tubular lining 134 is twisted, the lumen 132 is made smaller, thereby forming a closed configuration. The handle of a hand instrument may be inserted into the lumen 132 when it is in the open configuration, and as the tubular lining 134 is twisted, its walls are tightened around the handle to form a closed configuration adapted for holding onto the handle. The flexible and twistable tubular lining 134 is preferably made from an elastomeric material, and may be designed with a surface roughness, e.g., small protrusions or dots, to increase its surface friction. An example of a tubular lining 134 may be seen in Figure 9, where it is provided with end flanges 135 adapted for being fastened to the inner housing 120. The tubular lining 134 is here made in one piece.

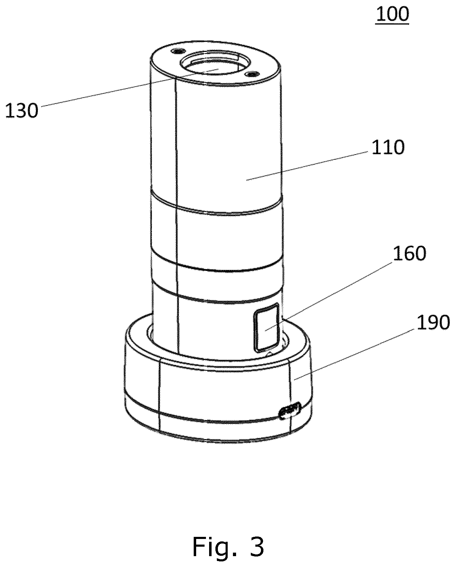

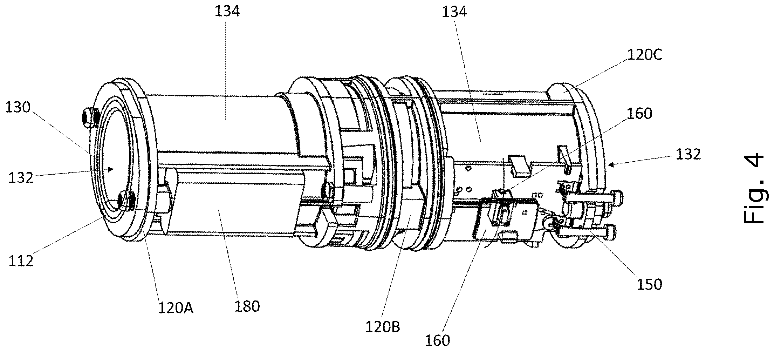

Figure 2 shows a grip aid device 100 in accordance with various embodiments of the invention, and in Figure 3, it is positioned in a charging dock 190. In Figure 4, the outer casing/housing 110 is removed to better show the inner casing/housing 120 and the inner lining 134.

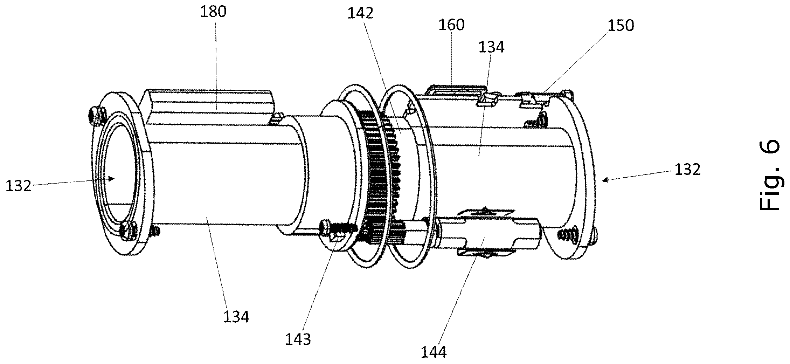

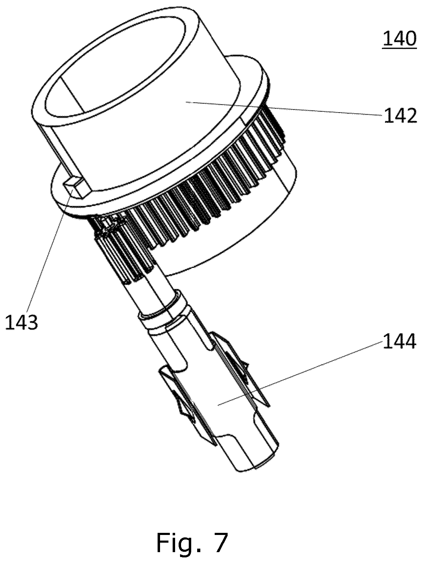

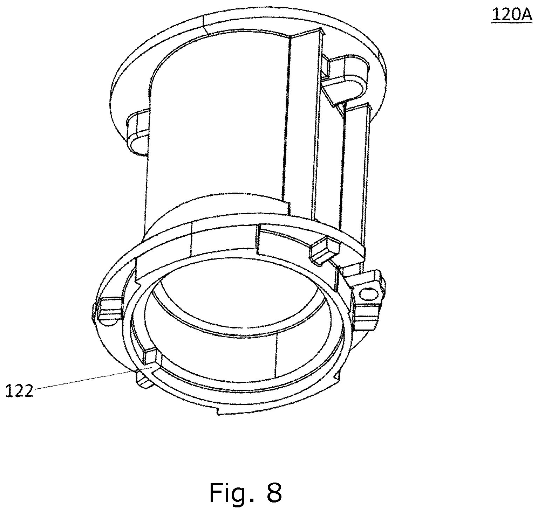

A twisting mechanism 140 (see e.g., Figure 7) is needed that is adapted for changing the configuration of the holder's lumen 132 from an open configuration to a closed configuration, and vice versa. This mechanism may take different forms, e.g., embodied with one or several twisting points, a single or multiple segments of the tubular lining 134 may be twisted, and with different areas of the tubular lining 134 fastened to the inner housing, preferably along the tubular lining's longitudinal axis. In the shown exemplary embodiment, the said twisting mechanism 140 comprises a ring- or tube-shaped drive shaft 142 rotatably mounted in the inner housing 120, and an electric motor unit 144 positioned mounted to the inner housing 120 (see Figure 6), directly, adapted to transfer a torque to the drive shaft 142. The drive shaft 142 and inner housing 110 is also shown comprising a stop mechanism adapted for allowing said drive shaft 142 to rotate a predefined number of degrees around its axis of rotation. The stop mechanism may be configured in many ways (e.g., by use of stop pins alone or stop pins and guide recesses) but is here exemplified comprising a first stop pin 143 extending outwardly from the drive shaft 142 (see Figures 6 and 7), and with a second stop pin 122 attached to the inner housing 120 (see Figure 8).

### References

- 10: Hand instrument
- 100: Grip aid device
- 110: Outer housing
- 112: Opening
- 114: Opening
- 120: Inner housing
- 122: Stop pin
- 130: Holder
- 132: Lumen
- 134: Lining
- 135: End flange
- 140: Twisting mechanism
- 142: Drive shaft
- 143: Stop pin
- 144: Motor unit
- 150: Contact
- 160: Activator
- 170: Control unit
- 180: Battery unit
- 190: Charging dock

## Claims

1. A hand-held grip aid device (100) for hand instrument manipulation, the grip aid device comprising:
- a housing (110, 120);
- a handle, optionally being part of said housing (110, 120); and
- a holder (130) integrated into or positioned within said housing (110, 120) and adapted for receiving the handle of a hand instrument;
wherein the holder (130) comprises:
- a lumen (132) adapted for being in an open configuration adapted for receiving the handle of a hand instrument, and in a closed configuration adapted for holding onto and into the lumen (132) inserted handle of a hand instrument;
- a tubular lining (134), preferably made from an elastomeric material, said lining (134) defining at least a part of said lumen (132);
**characterised by**
- a twisting mechanism (140) adapted for changing the configuration of said lumen (132) from said open configuration to said closed configuration by twisting said tubular lining (134), preferably around the tubular lining's (134) longitudinal axis.

2. The grip aid device (100) according to claim 1, wherein said twisting mechanism (140) comprises:
- a ring- or tube-shaped drive shaft (142) rotatably mounted in said housing (110, 120); and
- an electric motor unit (144) positioned within and/or mounted to said housing (110, 120), and indirectly, e.g., through a gear, or directly, adapted to transfer a torque to said drive shaft (142).

3. The grip aid device (100) according to claim 2, wherein said drive shaft (142) and said housing (110, 120) comprises a stop mechanism adapted for allowing said drive shaft (142) to rotate a predefined number of degrees around its axis of rotation.

4. The grip aid device (100) according to claim 3, wherein said stop mechanism comprises a first stop pin (143) extending outwardly from the drive shaft (142), and preferably a second stop pin (122) attached to said housing (120).

5. The grip aid device (100) according to any one of the claims 2-4, wherein said tubular lining (134) is made in one piece.

6. The grip aid device (100) according to any one of the claims 2-5, wherein said drive shaft (142) comprises a lumen, and wherein said tubular lining (134) is attached to the lumen of said drive shaft (142).

7. The grip aid device (100) according to claim 6, wherein said drive shaft (142) is positioned centrally along the length of said grip aid device (100).

8. The grip aid device (100) according to any one of the claims 1-7, wherein the lumen (132) of said holder (130) extends along the entire length of said holder (130), and wherein the lumen (132) of said holder (130) is open-ended, thereby allowing the handle of a hand instrument to be inserted there through.

9. The grip aid device (100) according to any one of the claims 1-7, the grip aid device (100) comprising an inner housing (120) and an outer housing (110), wherein said holder (130) is integrated into said inner housing (120), wherein said outer housing (110) is configured as a cover releasably fastened to said inner housing (120), and wherein said outer housing (110) comprises an opening (112) leading into the lumen (132) of said holder (130).

10. The grip aid device (100) according to claim 9, wherein the lumen (132) of said holder (130) extends along the entire length of said holder (130), wherein the lumen (132) of said holder (130) is open-ended, and wherein said outer housing (110) comprises openings (112, 114) leading into each of the lumen's ends, thereby allowing the handle of a hand instrument to be inserted there through.

## Patentansprüche

1. Handgehaltene Greifhilfevorrichtung (100) für eine Betätigung von Handinstrumenten, die Greifhilfevorrichtung umfassend:
- ein Gehäuse (110, 120);
- einen Griff, der optional Teil des Gehäuses (110, 120) ist; und
- einen Halter (130), der in das Gehäuse (110, 120) integriert oder in diesem positioniert ist und zum Aufnehmen des Griffs eines Handinstruments angepasst ist;
wobei der Halter (130) umfasst:
- ein Lumen (132), das angepasst ist, um in einer offenen Konfiguration, die zum Aufnehmen des Griffs eines Handinstruments angepasst ist, und in einer geschlossenen Konfiguration zu sein, die zum Halten des in das Lumen (132) eingeführten Griffs eines Handinstruments und zum Festhalten davon angepasst ist;
- eine rohrförmige Auskleidung (134), vorzugsweise aus einem elastomeren Material hergestellt, wobei die Auskleidung (134) mindestens einen Teil des Lumens (132) definiert;
**gekennzeichnet durch**
- einen Verdrehmechanismus (140), der zum Ändern der Konfiguration des Lumens (132) von der offenen Konfiguration zu der geschlossenen Konfiguration durch Verdrehen der rohrförmigen Auskleidung (134), vorzugsweise um die Längsachse der rohrförmigen Auskleidung (134), angepasst ist.

2. Greifhilfevorrichtung (100) nach Anspruch 1, wobei der Verdrehmechanismus (140) umfasst:
- eine ring- oder rohrförmige Antriebswelle (142), die in dem Gehäuse (110, 120) drehbar gelagert ist; und
- eine Elektromotoreinheit (144), die innerhalb des Gehäuses (110, 120) positioniert und/oder daran gelagert ist und indirekt, z. B. über ein Getriebe, oder direkt angepasst ist, um ein Drehmoment auf die Antriebswelle (142) zu übertragen.

3. Greifhilfevorrichtung (100) nach Anspruch 2, wobei die Antriebswelle (142) und das Gehäuse (110, 120) einen Anschlagmechanismus umfassen, der zum Ermöglichen der Antriebswelle (142), sich um eine vordefinierte Anzahl von Grad um ihre Drehachse zu drehen, angepasst ist.

4. Greifhilfevorrichtung (100) nach Anspruch 3, wobei der Anschlagmechanismus einen ersten Anschlagstift (143), der sich von der Antriebswelle (142) nach außen erstreckt, und vorzugsweise einen zweiten Anschlagstift (122) umfasst, der an dem Gehäuse (120) befestigt ist.

5. Greifhilfevorrichtung (100) nach einem der Ansprüche 2 bis 4, wobei die rohrförmige Auskleidung (134) einstückig hergestellt ist.

6. Greifhilfevorrichtung (100) nach einem der Ansprüche 2 bis 5, wobei die Antriebswelle (142) ein Lumen umfasst, und wobei die rohrförmige Auskleidung (134) an dem Lumen der Antriebswelle (142) befestigt ist.

7. Greifhilfevorrichtung (100) nach Anspruch 6, wobei die Antriebswelle (142) zentral entlang der Länge der Greifhilfevorrichtung (100) positioniert ist.

8. Greifhilfevorrichtung (100) nach einem der Ansprüche 1 bis 7, wobei sich das Lumen (132) des Halters (130) entlang der gesamten Länge des Halters (130) erstreckt, und wobei das Lumen (132) des Halters (130) offen ist, wodurch ermöglicht wird, dass der Griff eines Handinstruments dort hindurch eingeführt werden kann.

9. Greifhilfevorrichtung (100) nach einem der Ansprüche 1 bis 7, die Greifhilfevorrichtung (100) umfassend ein Innengehäuse (120) und ein Außengehäuse (110), wobei der Halter (130) in das Innengehäuse (120) integriert ist, wobei das Außengehäuse (110) als eine an dem Innengehäuse (120) lösbar angebrachte Abdeckung konfiguriert ist, und wobei das Außengehäuse (110) eine Öffnung (112) umfasst, die in das Lumen (132) des Halters (130) führt.

10. Greifhilfevorrichtung (100) nach Anspruch 9, wobei sich das Lumen (132) des Halters (130) entlang der gesamten Länge des Halters (130) erstreckt, wobei das Lumen (132) des Halters (130) offen ist, und wobei das Außengehäuse (110) Öffnungen (112, 114) umfasst, die in jedes der Enden des Lumens führen, wodurch ermöglicht wird, dass der Griff eines Handinstruments dort hindurch eingeführt werden kann.

## Revendications

1. Dispositif d'aide à la préhension tenu à la main (100) pour la manipulation d'instruments à main, le dispositif d'aide à la préhension comprenant :
- un boîtier (110, 120) ;
- une poignée, faisant éventuellement partie dudit boîtier (110, 120) ; et
- un support (130) intégré dans ledit boîtier (110, 120) ou positionné à l'intérieur de celui-ci et adapté pour recevoir le manche d'un instrument à main ;
dans lequel le support (130) comprend :
- une lumière (132) adaptée pour être dans une configuration ouverte adaptée pour recevoir le manche d'un instrument à main, et dans une configuration fermée adaptée pour maintenir le manche d'un instrument à main inséré sur et dans la lumière (132) ;
- une doublure tubulaire (134), de préférence fabriquée à partir d'un matériau élastomère, ladite doublure (134) définissant au moins une partie de ladite lumière (132) ;
**caractérisé par**
- un mécanisme de torsion (140) adapté pour changer la configuration de ladite lumière (132) de ladite configuration ouverte à ladite configuration fermée par torsion de ladite doublure tubulaire (134), de préférence autour de l'axe longitudinal de la doublure tubulaire (134).

2. Dispositif d'aide à la préhension (100) selon la revendication 1, dans lequel ledit mécanisme de torsion (140) comprend :
- un arbre d'entraînement (142) en forme d'anneau ou de tube monté de manière rotative dans ledit boîtier (110, 120) ; et
- une unité de moteur électrique (144) positionnée à l'intérieur dudit boîtier (110, 120) et/ou montée sur celui-ci, et adaptée indirectement, par exemple, par le biais d'un engrenage, ou directement, pour transmettre un couple audit arbre d'entraînement (142).

3. Dispositif d'aide à la préhension (100) selon la revendication 2, dans lequel ledit arbre d'entraînement (142) et ledit boîtier (110, 120) comprennent un mécanisme de blocage adapté pour permettre audit arbre d'entraînement (142) de tourner d'un nombre prédéfini de degrés autour de son axe de rotation.

4. Dispositif d'aide à la préhension (100) selon la revendication 3, dans lequel ledit mécanisme de blocage comprend une première goupille de blocage (143) s'étendant vers l'extérieur depuis l'arbre d'entraînement (142), et de préférence une seconde goupille de blocage (122) fixée audit boîtier (120).

5. Dispositif d'aide à la préhension (100) selon l'une quelconque des revendications 2 à 4, dans lequel ladite doublure tubulaire (134) est réalisée en une seule pièce.

6. Dispositif d'aide à la préhension (100) selon l'une quelconque des revendications 2 à 5, dans lequel ledit arbre d'entraînement (142) comprend une lumière, et dans lequel ladite doublure tubulaire (134) est fixée à la lumière dudit arbre d'entraînement (142).

7. Dispositif d'aide à la préhension (100) selon la revendication 6, dans lequel ledit arbre d'entraînement (142) est positionné au centre le long de la longueur dudit dispositif d'aide à la préhension (100).

8. Dispositif d'aide à la préhension (100) selon l'une quelconque des revendications 1 à 7, dans lequel la lumière (132) dudit support (130) s'étend sur toute la longueur dudit support (130), et dans lequel la lumière (132) dudit support (130) est ouverte aux extrémités, permettant ainsi au manche d'un instrument à main d'être inséré à travers celle-ci.

9. Dispositif d'aide à la préhension (100) selon l'une quelconque des revendications 1 à 7, le dispositif d'aide à la préhension (100) comprenant un boîtier interne (120) et un boîtier externe (110), dans lequel ledit support (130) est intégré dans ledit boîtier interne (120), dans lequel ledit boîtier externe (110) est conçu comme un moyen de recouvrement fixé de manière amovible audit boîtier interne (120), et dans lequel ledit boîtier externe (110) comprend une ouverture (112) menant dans la lumière (132) dudit support (130).

10. Dispositif d'aide à la préhension (100) selon la revendication 9, dans lequel la lumière (132) dudit support (130) s'étend sur toute la longueur dudit support (130), dans lequel la lumière (132) dudit support (130) est ouverte aux extrémités, et dans lequel ledit boîtier externe (110) comprend des ouvertures (112, 114) menant dans chacune des extrémités de la lumière, permettant ainsi au manche d'un instrument à main d'être inséré à travers celle-ci.
